(19) Europäisches Patentamt
European Patent Office
Office européen des brevets

(11) Publication number: **0 413 839 A1**

# (12) EUROPEAN PATENT APPLICATION

(21) Application number: 89115466.8

(51) Int. Cl.[5]: C07K 7/10, A61K 37/02

(22) Date of filing: 22.08.89

(43) Date of publication of application: 27.02.91 Bulletin 91/09

(84) Designated Contracting States: DE FR GB IT NL

(71) Applicant: THE ADMINISTRATORS OF THE TULANE EDUCATIONAL FUND
1430 Tulane Avenue
New Orleans, LA 70112(US)

(72) Inventor: Schally, Andrew V.
5025 Kawanne Avenue
Metairie, LA 70002(US)
Inventor: Bajuz, Sander
P.O. Box 82
Budapest(HU)
Inventor: Zarandi, Marta
420 Hessmer Avenue
Metairie LA 70002(US)

(74) Representative: Dr. Fuchs, Dr. Luderschmidt Dipl.-Phys. Seids, Dr. Mehler Patentanwälte
Abraham-Lincoln-Strasse 7
D-6200 Wiesbaden(DE)

## (54) GHRH analogs.

(57) There is provided a novel series of synthetic GHRH analog peptides which are extremely potent in stimulating the release of pituitary GH in animals, including humans, which are resistant to enzymatic degradation in the body in view of the provision of the omega-guanidino lower alkyl group at the terminal 28-position of the peptide.

EP 0 413 839 A1

# GHRH ANALOGS

## FIELD OF THE INVENTION

The present invention relates to peptides having influence on the function of the pituitary gland in humans and other animals. In particular, the present invention is directed to a peptide which promotes the release of growth hormone by the pituitary gland.

## BACKGROUND OF THE INVENTION

Physiologists have long recognized that the hypothalamus controls the secretory functions of the adenohypophysis with the hypothalamus producing special substances which stimulate or inhibit the secretion of each pituitary hormone. Hypothalamic releasing factors have been characterized for the pituitary hormone thyrotropin (the tripeptide TRF), for the pituitary gonadotropins luteinising hormone and follicle stimulating hormone (the decapeptide LRF, LH-RH or GnRH) and for the pituitary hormone and adreno-corticotropin (the 41-amino acid polypeptide CRF). An inhibitory factor, called somatostatin, has also been characterized in the form of a tetradecapeptide which inhibits the secretion of growth hormone (GH). GH releasing factors (GRFS) have been isolated from human pancreatic tumor as well as rat, porcine, bovine, ovine, caprine and human hypothalami. With the exception of the rat, all characterized GRFs contain 44 amino acids with an amidated carboxy-terminus. Each of these hypophysiotropic factors has been reproduced by total synthesis. Analogs of the native structures have also been synthesized in order to elucidate structure-activity relationships and, eventually, to provide synthetic congoners with improved properties, such as increased hormonal activity and/or metabolic stability. Studies with the synthetic human growth hormone releasing factor (hGRF) and its analogs (N. Ling., et al, Biochem. Biophys. Res. Commun., (1984), vol. 122, pp. 304-310; vol. 123, pp. 854-861; V. A. Lance, et al., Biochem. Biophys. Res. Commun., 1984, vol. 119, pp. 165-272)) have revealed that

a) deletion of the $NH_2$-terminal tyrosine residue of hGRF causes its activity to drop to 0.1%; N-acetylation of this residue or change of replacement of L by D isomers lowers the in vitro bioactivity of hGRF (1-40)-OH to 2-3%, and causes a 10-12 fold increase in the in vivo potency of hGRF(1-29)-$NH_2$; these findings indicate that the presence of said residue is essential for imparting the hGRF molecule with high bioactivity;

b) fragments containing the first 29 residues at least and being terminated with an amide group, e.g., hGRF(1-29)-$NH_2$ and hGRF(1-37)-$NH_2$, have at least 50% of the potency of hGRF; removal of the C-terminal amide group or further deletion of amino acids results in a marked decrease in bioactivity even if the C-terminus is amidated, e.g., hGRF(1-29)OH, hGRF(1-27)-$NH_2$ and hGRF(1-23)-$NH_2$ exhibit 12.6%, 1.12% and 0.24% of the potency of hGRF, respectively; these findings indicate the significance of the arginine amide, i.e., -Arg-$NH_2$ residue at position 29 of hGRF.

With respect to deamidation of the C-terminal carboxamide function, e.g., transformation of hGRF(1-29)-$NH_2$ into hGRF(1-29)OH, which can easily occur by the action of hydrolytic enzymes under physiological conditions, and which is accompanied with substantial loss of bioactivity, we may take into consideration our previous observations that the 4-guanidino-butylamino group, the so-called agmatine (Agm) residue, can play the role of the arginine residue in certain tripeptide inhibitors of trypsin-like enzymes and provide resistance to enzymatic degradation (S. Bajusz, et al., in : PEPTIDES, 1982, (K. Blaha and P. Melon, eds.), Walter Gruyter, Berlin-New York, 1983, pp. 643-647). Classical (solution) methods have only been employed for the preparation of agmatine peptides to date; it is desired to provide methodology for the solid phase synthesis of these peptides using techniques of peptide synthesis which have proven to be suitable for preparing GRFs and their analogs.

## SUMMARY OF THE INVENTION

There is provided a novel series of synthetic peptides which are extremely potent in stimulating the release of pituitary GH in animals, including humans, which are resistant to enzymatic degradation in the

body in view of the provision of an omega-guanidino lower alkyl group at the terminal 28-position of the peptide. There are also provided several methods of attaching said omega- guanidino alkyl group to a support phase, procedures for building up the aforesaid peptide by means of the solid phase synthesis and procedures for cleaving said peptide from said support phase. The ultimate products of the present invention are peptides (abbreviated [PeP]) having the sequence:

$Q^1$-CO-$R_2$-$R_3$-$Ala_4$-$Ile_5$-$Phe_6$-$Thr_7$-$R_8$-$Ser_9$-$R_{10}$-$Arg_{11}$-$R_{12}$-$R_{13}$-$R_{14}$-$R_{15}$-$Gln_{16}$-$R_{17}$-$R_{18}$-$Ala_{19}$-$Arg_{20}$-$R_{21}$-$R_{22}$-$R_{23}$-$R_{24}$-$R_{25}$-$Ile_{26}$-$R_{27}$-$R_{28}$-NH-$Q^2$ I.

wherein $Q^1$ is 3-carboxypropyl, or a 2-carboxybenzamidomethyl group, or an omega or alpha-omega substituted alkyl of the structure:

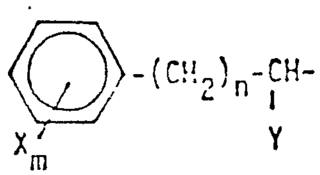

II.

where:
x = H,OH
Y = H,OH,$NH_2$,$CH_3CONH$,
m = 1,2
n = 0,1,2
$R_2$ is Ala or D-Ala
$R_3$ is Asp, D-Asp, Glu or D-Glu
$R_8$ is Asn, D-Asn, Ser or D-Ser
$R_{10}$ is Tyr or D-Tyr
$R_{12}$ is Lys, D-Lys, Arg, Orn, D-Arg or D-Orn
$R_{13}$ is Val or Ile
$R_{14}$ is Leu or D-Leu
, $R_{15}$ is Gly or Ala, preferably Ala
$R_{17}$ is Leu or D-Leu
$R_{18}$ is Tyr or Ser
$R_{21}$ is Lys, D-Lys, Orn, D-Orn, Arg and D-Arg
$R_{22}$ is Leu, D-Leu or Ala
$R_{23}$ is Leu or D-Leu
$R_{24}$ is Gln or His
$R_{25}$ is Asp, D-Asp, Glu or D-Glu
$R_{27}$ is Met, D-Met, Ala, Nle, Ile, Val, Nva, Leu,preferably Nle
$R_{28}$ is Asn or Ser
$Q^2$ is a lower omega-guanidino-alkyl group having a formula:

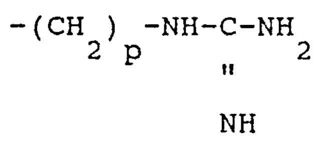

(III)

wherein p = 2 - 6
and the pharmaceutically acceptable salts addition thereof with the pharmaceutically acceptable organic or inorganic bases and organic or inorganic acids.

The peptides of the present invention [PeP] are synthesized in accordance with the following procedures.

The first method of $NH_2$-$Q^2$ moiety comprises the sequential steps of reacting a tert-butyloxycarbonylating agent with $NH_2$-$Q^2$ in the presence of a base to form Boc- NH-$Q^2$ (IV), reacting said Boc-NH-$Q^2$ with an arylsulphonyl halide suitably of the formula:

Hal-$SO_2$-< o >-O-$CH_2$-COOH (V),

wherein Hal is chloro or bromo, and < o > signifies (throughout the application) an unsubstituted benzene ring,

at between -5 and 10°C in a water soluble solvent in the presence of aqueous alkali to form the corresponding

$Boc\text{-}, NH\text{-}Q^2,SO_2\text{-}< o > \text{-}O.CH_2\text{-}COOH$ (VI)

wherein $Q^{2'}$ is $-(CH_2)_p-NH-C(NH_2)=N-$ (VII)

which is coupled to the support phase [SP] by the action of a diloweralkyl carbodiimide to yield:

$Boc\text{-}NH\text{-}Q^{2'}\text{-}SO_2\text{-}< o >\text{-}O\text{-}CH_2\text{-}CO\text{-}$ [SP] (VIII)

It is generally preferred that the support phase [SP] is an amino type resin of the formula

$H_2N\text{-}CH_2\text{-}< o >$-"[Pm] (IX)"

where [Pm] is a polymeric substrate.

This approach contemplates building the peptide sequence by the Boc protocol which is discussed in detail hereinbelow.

Another embodiment is available for attaching the guanidino alkyl group to the support phase. This method comprises the sequential steps of reacting N-benzyloxy carbonyl chloride with $NH_2\text{-}Q^2$ in the presence of a base to form $Cbz\text{-}NH\text{-}Q^2$, reacting said $Cbz\text{-}NH\text{-}Q^2$ with a substituted arylsulphonyl halide of the formula:

$$Hal\text{-}SO_2\text{-}< \ o \ >\text{-}O\text{-}CH_2\text{-}COOH \quad (X)$$
$$/$$
$$M_s$$

wherein Hal is chloro or bromo , M is lower alkyl or lower alkoxy of 1 to 5 carbon atoms, and is 1-3, in the presence of a strong aqueous base and removing the Cbz group by catalytic hydrogenation to yield

$$H_2N\text{-}Q^{2'}\text{-}SO_2\text{-}< \ o \ >O\text{-}CH_2\text{-} \ COOH \quad (XI)$$
$$/$$
$$M_s$$

reacting this product with 9-fluorenylmethoxy carbonyl chloride in the presence of a base to yield

$$Fmoc\text{-}NH\text{-}Q^{2'}\text{-}SO_2\text{-}< \ o \ >\text{-}OCH_2\text{-} \ COOH \quad (XII)$$
$$/$$
$$M_s$$

coupling this product to said support phase [SP] to yield:

$$Fmoc\text{-}NH\text{-}Q^{2'}\text{-}SO_2\text{-}< \ o \ >\text{-}O\text{-}CH_2\text{-}CO\text{-} \ [SP] \quad (XIII)$$
$$/$$
$$M_s$$

As indicated, this procedure is preferred when it is intended to use the Fmoc protocol which is also set forth in detail hereinbelow.

Utilizing the aforementioned protected amino-alkyl-guanidino sulfophenoxy-acetyl support phase as the terminal, the desired peptide itself is built up in the conventional manner for solid phase synthesis utilizing

the protective groups and the specific procedures set forth in detail hereinbelow. When the synthesis is complete, the guanidino alkyl end of the peptide is cleaved from the sulfonyl group to which it is attached by one or two procedures. If the Boc protocol is utilized then the cleaving agent is anhydrous hydrofluoric acid, on the other hand, in the Fmoc protocol the cleaving agent is trifluoroacetic acid.

The pharmaceutical compositions in accordance with the invention include the peptides of Formula I, or a nontoxic salt of any of these, dispersed in a pharmaceutically or veterinarily acceptable liquid or solid carrier. Such pharmaceutical compositions can be used in clinical medicine, both human and veterinary, for administration for therapeutic purposes, and also diagnostically. Moreover, they can be used to promote the growth of warm-blooded animals, while improving feed utilization, increasing lean/fat ratio favoring muscle gain at the cost of fat, and also to enhance milk production in lactating cattle.

## DESCRIPTION OF THE PREFERRED EMBODIMENTS

The nomenclature used to define the peptides is that specified by the IUPAC-IUB Commission on Biochemical Nomenclature ( European J. Biochem., 1984, 138 , 9-37), wherein in accordance with conventional representation the amino groups at the N-terminus appears to the left and the carboxyl group at the C-terminus to the right. By natural amino acid is meant one of common, naturally occurring amino acids found in proteins comprising Gly, Ala, Val, Leu, Ile, Ser, Thr, Lys, Arg, Asp, Asn, Glu, Gln, Cys, Met, Phe, Tyr, Pro, Trp and His. By Nle is meant norleucine, by Nva is meant norvaline and by MeAla is meant N-methyl-alanine. When the amino acid residue has isomeric forms, it is the L-form of the amino acid that is represented unless otherwise expressly indicated.

Other abbreviations used are:

| | |
|---|---|
| AcOH | acetic acid |
| AcOEt | ethyl acetate |
| $Ac_2O$ | acetic anhydride |
| Boc- | tert-butyloxycarbonyl- |
| DIC | diisopropylcarbodiimide |
| Agm | agmatine |
| DCM | dichloromethane |
| Chx | cyclohexyl- |
| DIEA | diisopropylethylamine |
| DMF | dimethylformamide |
| HOBt | 1-hydroxybenzenetriazole hydrate |
| HPLC | high performance liquid chromatography |
| Fmoc- | fluorenylmethyoxycarbonyl- |
| MeOH | methyl alcohol |
| TEA | triethylamine |
| DCCI | dicyclohexylcabodiimide |
| 2-Cl-Cbz | 2-chloro-benzyloxycarbonyl- |
| DCB | 2,6-dichlorobenzyl- |
| Tos | p-toluenesulfonyl- |
| TFA | trifluoroacetic acid |
| Cbz | benzyloxycarbonyl- |

The procedures of the present invention may be carried out using a variety of support phases. These support phases may be amino or hydroxy resins such as aminomethyl resins (suitably 1% cross linked), benzhydrylamine resins (suitably 2% cross linked), p-methylbenzhydrylamine resins (suitably 2% cross linked) and the like.

As starting material in the process, there may be utilized any amino lower alkyl guanidine having between 2 and 6, suitably 3 thru 5 carbon atoms in the alkyl moiety. Especially preferred however is agmatine or 4-guanidino butylamine.

The key to the success of the present invention is the provision of a stable but readily cleavable bridging group between the guanidino moiety and the support phase. It has been found that such a bridge

may be readily provided by the sulfonyl phenoxy acetyl moiety.

In one embodiment of the synthesis, the primary amino group of agmatine is protected with a tert-butyloxy carbonylating agent, suitably ditert-butyl dicarbonate, by reaction preferably at ambient temperatures in the presence of a base, providing the protected material (V) which is then reacted in a similar solvent system with the arylsulfonyl halide (V) which may be a sulfonyl chloride or sulfonyl bromide. Where the ultimate synthetic sequences intended to follow the Boc protocol, the aromatic ring of the arylsulfonyl halide is not substituted since such a situation will permit the use of trifluoroacetic acid as the intermediate deprotecting agent and hydrogen fluoride as the agent for final cleavage from the resin. The thus obtained protected aminoalkyl guanidino-sulfonyl phenoxyacetic acid (VI) is then coupled to the support phase. In the preferred procedure, the coupling is carried out using any of the known dialkyl carbodiimide coupling procedures. For example, there may be utilized diisopropyl-carbodiimide in the presence of 1-hydroxybenzenetriazole hydrate in dimethylformamide at ambient temperatures. Alternatively, there may be employed N,N′dicyclohexyl-carbodiimide. In this procedure the aforesaid Boc-aminoalkyl guanidino-sulfonyl-phenoxyacetic acid (VI) is mixed with the carbodiimide in 2 to 1 molar ratio, the formed N,N′dicyclohexyl urea is removed by filtration and the resulting solution is added to the support phase, suitably to an aminomethyl resin.

As previously stated, if the protecting group is Boc, the aryl moiety of the sulfonamide bridge is unsubstituted. However, where it is desired to proceed under the Fmoc protocol, it is desirable to substitute the aforesaid phenyl moiety with up to three methyl or methoxy groups since these weaken the sulfonamide group and enable it to be cleaved by trifluoroacetic acid.

In this second embodiment in place of utilizing Boc to protect the aminoalkyl guanidino moiety, there is utilized N-benzyloxycarbonyl chloride in the presence of an aqueous alkali, suitably sodium hydroxide, preferably at about 4N under agitation and cooling to between about 5 and about 15° C. Upon acidification the protected product is precipitated and thereafter is reacted with the arylsulfonyl chloride (X) as previously. However, as stated above, in this case the arylsulfonyl chloride (X) should preferably carry between 1 and 3 substituents which may be alkyl or lower alkoxy, suitably methyl or methoxy. This reaction is carried out in the presence of a strong but dilute aqueous base, suitably about 1N sodium hydroxide. The reaction mixture is acidified and the product extracted, suitably with a water immiscible organic solvent such as ethyl acetate. The extract is then concentrated and hydrogenated, suitably at atmospheric pressure and room temperature in the presence of a catalyst such as palladium on charcoal in a reduction inert solvent. If ethyl acetate is used as the extractant, this may be used as the hydrogenation solvent. After removal of the Cbz group, the product (XI) is reacted with Fmoc-Cl in the presence of a base to yield the corresponding Fmoc protected aminoalkyl guanidino-sulfonyl aryloxy acetic acid (XII) which is then coupled to the support phase in the manner set forth hereinabove.

The peptides are synthesized by a suitable method, such as by exclusively solid phase techniques, by partial solid-phase techniques, by fragment condensation or by classical solution phase synthesis. The employment of recently developed recombinant DNA techniques may be used to prepare a portion of an analog containing only natural amino acid residues. For example, the techniques of exclusively solid-phase synthesis are set forth in the textbook "Solid Phase Peptide Synthesis", J. M. Stewart and J. D. Young, Pierce Chem. Company, Rockford, Ill., 1984 (2nd. ed.), G. Barany and R.B. Merrifield, "The Peptides", Ch. 1, 1-285, pp. 1979, Academic Press, Inc., and M. Bodanszky, "Principles of Peptide Synthesis", Springer-Verlag, 1984. Classical solution synthesis is described in detail in the treatise "Methoden der Organischen Chemie (Houben-Weyl): Synthese von Peptiden", E. Wunsch (editor) (1974) Georg Thieme Verlag, Stuttgart, W. Germany.

Common to such synthesis is the protection of the reactive side chain functional groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting groups to allow subsequent reaction to take place at that location. Accordingly, it is common that, as a step in the synthesis, an intermediate compound is produced which includes each of the amino acid residues located in its desired sequence in the peptide chain with side-chain protecting groups linked to the appropriate residues.

Also considered to be within the scope of the present invention are intermediates of the formula:

[PR][PeP] attached to a support phase [SP] selected from the group consisting of amino type resins, said combination having the structure:

$$[PR][PeP]\ -SO_2-\langle\ o\ \rangle-O-CH_2-CO-\ [SP]$$
$$/$$
$$M_s$$

where M is hydrogen, lower alkyl or lower alkoxy of 1 to 5 carbon atoms, suitably methyl or methoxy, s is 0, 1, 2 or 3, [SP] is an amino type resin, suitably [PR] [PeP] has the structure

$X^1$-$Q^1$-CO-$R_2$-$R_3$($X^3$)-$Ala_4$-$Ile_5$-$Phe_6$-$Thr_7$($X^7$)-$R_8$($X^7$ or H)-$Ser_9$($X^7$)-$R_{10}$($X^{10}$)-$Arg_{11}$($X^{11}$)-$R_{12}$($X^{11}$ , $X^{12}$)-$R_{13}$-$R_{14}$-$R_{15}$-$Gln_{16}$-$R_{17}$-$R_{18}$($X^7$ or $X^{10}$)-$Ala_{19}$-$Arg_{20}$($X^{11}$)-$R_{21}$($X^{12}$)-$R_{22}$-$R_{23}$-$R_{24}$(H or $X^{24}$)-$R_{25}$($X^3$)-$Ile_{26}$-$R_{27}$- $R_{28}$(H or $X^7$ )-$NH_2$-$Q^{2'}$     IA

wherein: $X^1$ is either hydroxyl or an amino protecting group or nil, depending on the meaning of Y in structure II.

$X^3$ is a suitable ester-forming protecting group for the carboxyl group of Asp or Glu. In the Boc protocol, cyclohexyl esters; in the Fmoc protocol tert-butyl esters are preferred.

$X^7$ may be a suitable protecting group for the hydroxyl group of Thr or Ser, such as tert-butyl, Bzl, and 2,6-dichloro-benzyl. The preferred protecting in the Boc protocol is Bzl, and in the Fmoc protocol is tert-butyl.

$X^{10}$ may be a suitable protecting group for the phenolic hydroxyl group of Tyr, such as tert-butyl, Bzl, 4Br-Cbz and 2,6-dichloro benzyl (DCB). The preferred protecting group in the Boc protocol is 2,6-dichlorobenzyl, and in the Fmoc protocol is tert-butyl.

$X^{11}$ is a suitable protecting group for the guanidino group of Arg, such as nitro, Tos, 4-methoxy-2,3,6-trimethylbenzenesulfonyl; in the Boc protocol Tos is the preferred group and 4-methoxy-2,3,6-trimethylbenzenesulfonyl in the Fmoc protocol.

$X^{12}$ is a suitable protecting group for the side chain amino group of Lys. Illustrative of suitable side chain amino protecting groups are 2-chlorobenzyloxycarbonyl (2-Cl-Z), benzyloxycarbonyl and in the Boc protocol 2-chlorobenzyloxycarbonyl is the preferred protecting group and in the Fmoc protocol Boc is thus utilized.

$X^{24}$ is hydrogen or a protecting group for the imidazole nitrogen of His, such as Tos.

The selection of a side chain amino protecting group is not critical except that generally one is chosen which is not removed during deprotection of the alpha-amino groups during the synthesis.

In selecting a particular side chain protecting group to be used in the synthesis of the peptides, the following general rules are followed: (a) the protecting group preferably retains is protecting properties and is not split off under coupling conditions, (b) the protecting group should be stable to the reagent, is preferably stable under the reaction conditions selected for removing the alpha-amino protecting group at each step of the synthesis, and (c) the side chain protecting group must be removable, upon the completion of the synthesis containing the desired amino acid sequence, under reaction conditions that will not undesirably alter the peptide chain.

The peptides are preferably prepared using solid phase synthesis, such as that generally described by Merrifield, J. Am. Chem. Soc. , 85 , p. 2149 (1963), although other equivalent chemical syntheses known in the art can also be used as previously mentioned.

$$Boc\text{- or }Fmoc\text{-}NH\text{-}Q^{2'}\ -SO_2-\langle\ o\ \rangle-O-CH_2-CO-[SP]\ \text{is}$$
$$/$$
$$M_s$$

prepared as set forth above and constitutes the starting material for the synthetic sequence.

After removal of the alpha-amino protecting group, the remaining alpha-amino- and side chain-protected amino acids are coupled step-wise in the desired order to obtain the intermediate compound defined hereinbefore, or as an alternative to adding each amino acid separately in the synthesis, some of them may be coupled to one another prior to addition to the solid phase reactor. The selection of an appropriate coupling reagent is within the skill of the art. Particularly suitable as a coupling reagent is N,N′-diisopropyl

carbodiimide (DIC).

Each protected amino acid or amino acid sequence is introduced into the solid phase reactor in about a threefold excess, and the coupling may be carried out in a medium or dimethylformamide (DMF): $CH_2Cl_2$ - (1:1) or in DMF or $CH_2Cl_2$ alone. In cases where incomplete coupling occurs, the coupling procedure is repeated before removal of the alpha-amino protecting group prior to the coupling of the next amino acid. The success of the coupling reaction at each stage of the synthesis, is preferably monitored by the ninhydrin reaction, as described by E. Kaiser, et al., Anal. Biochem. , 34 , 595 (1970).

After the desired amino acid sequence has been completed, the intermediate peptide can be removed from the resin support by treatment with a reagent such as liquid hydrogen fluoride or trifluoroacetic acid, which not only cleaves the peptide from the resin but also cleaves all remaining side chain protecting groups $X^3$, $X^7$, $X^{10}$, $X^{11}$, $X^{12}$, and $X^{24}$ and the anchoring guanidinosulfo bond, if present, and also the alpha-amino or alpha hydroxyl protecting group $X^1$. As indicated above these groups have different values depending on whether Boc or Fmoc protocols are used, to obtain the desired free peptide.

The products of the present invention may be utilized to enhance the milk production of females of milk producing mammals, suitably but not exclusively goats and cows, most preferrably cows. The compound of the present invention are suitably administered to the subject animals i.v, s.c. or i.m., the later two modes being preferred. Administration is in any physiologically acceptable injectable carrier, physiological saline being acceptable, though other carriers known to the art may also be used. The dosage level should be between 0.2 and 2 micrograms per kg bodyweight per injection. These dosage ranges are merely preferred. Administration may be between 1 and 4 times per day. It is convenient to inject the animal whenever it is milked. Other equivalent modes of administration are also within the scope of this invention, ie continuous drip, depot injections, time release modes such as micro capsules and the like. The following Examples set forth a preferred method for synthesizing peptides by the solid-phase technique.

## EXAMPLE I

### Synthesis of Boc-Agmatine (V)

To a stirred suspension of 19.56 g. (85.5 mmol) agmatine sulfate in 300 ml. 50% aqueous tetrahydrofuran containing 25 ml. (178.6 mmol) triethylamine, was added 25 g. di-tertbutyl dicarbonate. After 4 hours, an additional 12.5 g. of dicarbonate was added and after a second four hours, another 12.5 g., to give a total of 50.0 g. (229 mmol). Stirring was continued overnight, followed by filtration through a Buchner funnel to remove insoluble matter. The filtrate was transferred to a separatory funnel and the bottom aqueous layer was drawn off, this layer was extracted with 100 mol. of diethyl ether then lyophilized. The residue was dissolved in 200 ml. water. To this solution, 15 g. of sodium bicarbonate was added and stirred well while solids began to form, after chilling in an ice bath, the dry solids were collected to recover 15.8 g. product, m.p. 126-128 °C (dec.).

250 mg. of this material were dissolved in 2.5 ml. of hot water and 2.5 ml. of saturated sodium bicarbonate solution were added and kept in refrigerator overnight. 160 mg. of material were obtained. m.p. 134-136 °C (dec.).

15 g. of crude Boc-agmatine was taken up in 50 ml. hot water, chilled to room temperature and seeded with the purified material then placed in a refrigerator overnight. Yield 10.4 g., m.p. 134-135 °C (dec.). A second crop of 2.4 g., m.p. 134-135 °C (dec.) could be obtained by the addition of an equal volume of saturated sodium bicarbonate solution. Total yield is 12.9 g.

## EXAMPLE II

### Synthesis of 4-Chlorosulfonyl Phenoxyacetic Acid

50 mM (7, 6 g) phenoxyacetic acid was dissolved in 75 ml chloroform, cooled in ice-bath and stirred. 16.5 ml chlorosulfonic acid in 25 ml chloroform was added dropwise during a period of 10-15 minutes. The temperature was kept at 0° for 20 minutes then allowed to remain at room temperature for an additional 30

minutes. The two-phase liquid was poured into a beaker containing about 300 g. of crushed ice. The separated aqueous phase was extracted twice with 50 ml ether, then the combined organic phase was dried over anhydrous sodium sulfate. The solvent was removed in vacuo and the remaining material was recrystallized from ether-hexane mixture. Yield 9.75 g. (77.8%, Melting point: 155-157° (uncorrected).

TLC : Sigma $F_{254}$ anal. plate. Detection: UV 254 nm, 355 nm.
Eluent A) Ethyl Acetate - Acetic acid 9:1 v/v
Eluent B) Ether-Hexane 2:1 v/v plus one drop of acetic acid in 10 ml of eluent.
$R_f$ : Phenoxyacetic Acid,
System A: 0.85
System B: 0.34
$R_f$ : 4-Chlorosulfonyl Phenoxyacetic Acid,
System A: 0.77
System B: 0.17

In accordance with the above procedure but utilizing, in place of phenoxy acetic acid, 2,3,5-trimethyl phenoxy acetic acid and 2,3,6-trimethyl phenoxy acetic acid, there are obtained the corresponding 4-chlorosulfonyl-2,3,5-trimethyl phenoxy acetic acid ($R_f$ values: 0.78 and 0.75, respectively, in a 8:4:0.05 (v/v) mixture of ether-hexane-acetic acid on Sigma $F_{254}$ TLC plate).

## EXAMPLE III

### Boc-Agmatine-[SPA] (VII)

Boc-Agmatine (4.29 g, 10 mmole) is taken up in a mixture of acetone (50 ml) and 1N sodium hydroxide (50 ml) with stirring and cooling in an ice bath and 4-chlorosulfonyl phenoxyacetic acid (3.8 g, 15 mmole) added to the solution. Stirring is continued for 3 hours and the pH of the reaction mixture is kept at 11-12 by adding 4N sodium hydroxide. The reaction mixture is acidified with 10% citric acid solution and concentrated under reduced pressure to the half of its volume, and extracted with ethyl acetate (3 x 50 ml). The combined extracts are washed with saturated sodium chloride solution, dried over sodium sulfate, and evaporated under reduced pressure. Boc-agmatine-sulfonyl-phenoxyacetic acid (abbreviated as Boc-agmatine-[SPA]) is obtained as thick oil (3.6 g, 81%). $R_f$ = 0.5 in a 9:1 mixture (v/v) of ethyl acetate-acetic acid on Sigma $F_{254}$ TLC plate.

In accordance with the foregoing procedure but where, in place of Boc-agmatine, there is used Cbz-agmatine and in place of 4-chlorosulfonyl phenoxy acetic acid, there is utilized 4-chlorosulfonyl-2,3,5-trimethyl phenoxy acetic acid or 4-chloro-sulfonyl 2,3,6-trimethyl phenoxy acetic acid, there is obtained the corresponding Cbz-agmatine-2,3,5-trimethyl-[SPA] and Cbz-agmatine-2,3,6-trimethyl-[SPA].

## EXAMPLE IV

### Coupling of Boc-Agmatine-(SPA) to Support Phase

3.11 g (7 mM) Boc-Agmatine-(SPA) in 50 ml DMF was reacted with 1.89 g (14 mM) HOBt and 1.1 ml (7 mM) DIC at room temperature for 30 minutes, and the resulted active ester solution in this way was added to 5 g. aminomethyl-resin (0.7 m. equiv./g. capacity) which was swollen in dichloro-methane, deprotonated in 10% diisopropyl ethylamine chloroform mixture and washed twice with DMF. The reaction was complete for 90 minutes, tested by ninhydrin-reaction. Finally, the coupled resin was washed with DMF, dichloromethane and DMF again.

## EXAMPLE V

Synthesis of Cbz-Agmatine

Agmatine sulfate (2.28 g, 10 mM) and sodium hydrogencarbonate (2.52 g, 30 mM) was dissolved in a mixture of 20 ml water and 20 ml dioxane. The mixed solution was cooled on ice bath and benzyloxycarbonylchloride (1.85 ml, 13 mM) in 10 ml dioxane was added dropwise during a period of 15 minutes. The stirring was continued at 0 °C for 1 hour and overnight at room temperature. The reaction mixture was evaporated to dryness, the remaining solid material was agitated with 2 x 50 ml chloroform and after filtration, the solution was evaporated under reduced pressure. The oily compound was used without further purification.

$R_f$ : 0.66 in mixture of Ethyl acetate-Pyridine-Acetic Acid-Water 45:20:6:11 (v/v) on Sigma $F_{254}$ TLC plate. Detection: under UV light, Sakaguchi reagent

EXAMPLE VI

Synthesis of Agmatine-sulfonyl-2,3,5(and 2,3,6)-Trimethylphenoxy acetic acids

Cbz-agmatine (2.85 g, 10 mM) and 4-chlorosulfonyl-2, 3,5-trimethylphenoxy acetic acid (or 4-chlorosulfonyl-2,3, 6-trimethylphenoxy acetic acid (4.39 g, 15 mM) were reacted as described in Example III. Cbz-agmatine-sulfonyl-2,3,5 (or 2,3,6)-trimethylphenoxy acetic acid obtained was dissolved in ethanol (50 ml) and hydrogenated over palladium-charcoal catalyst (0.5 g). The catalyst was filtered off and the solvent was removed under reduced pressure. The residue was crystallized from an ethanol-water mixture to yield agmatine-sulfonyl-2,3,6-trimethylphenoxy acetic acid (2.70 g. 70% and 2.78 g. 72%, respectively).

$R_f$ values for both compounds were the same, 0.28 in a 4:1:1 (v.v) mixture of n-butanol-acetic acid-water on Sigma $F_{254}$ TLC plate.

EXAMPLE VII

Coupling of Fmoc-Agmatine-sulfonyl-2,3,5 (and 2,3,6)-trimethylphenoxy acetic acid to Support Phase

Agmatine-sulfonyl-2,3,5 (or 2,3,6)-trimethylphenoxy-acetic acid (2.70 g, 7 mM) was dissolved in a 10% solution of sodium carbonate (18.6 ml) and dioxane (11 ml) added. The solution was stirred and cooled in an ice-water bath, then 9-fluorenylmethyl chlorocarbonate (1.82 g, 7 mM) was added in small portions. The stirring was continued at ice-bath temperature for 4 hours and then at room temperature for a further 8 hours. The mixture was poured into 400 ml water and extracted with 2 x 100 ml ether. The aqueous phase was acidified with 1:1 hydrochloric acid solution and extracted 3 x 100 ml ethyl acetate. After drying over anhydrous sodium sulfate the solvent was removed under reduced pressure. The Fmoc-Agmatine-sulfonyl-2,3,5 (or 2,3,6)-trimethylphenoxy acetic acid obtained was dissolved in DMF (50 ml) and coupled to 5 g. aminomethyl resin as described in Example IV.

EXAMPLE VIII

The synthesis of an hGHRH analog of the formula:
3-(4-hydroxyphenyl)propionyl-$Ala_2$-$Asp_3$-$Ala_4$-$Ile_5$-$Phe_6$-$Thr_7$-$Asn_8$-$Ser_9$-$Tyr_{10}$-$Arg_{11}$-$Lys_{12}$-$Val_{13}$-$Leu_{14}$-$Gly_{15}$-$Gln_{16}$-$Leu_{17}$-$Ser_{18}$-$Ala_{19}$-$Arg_{20}$-$Lys_{21}$-$Leu_{22}$-$Leu_{23}$-$Gln_{24}$-$Asp_{25}$-$Ile_{26}$-$Nle_{27}$ -$Ser_{28}$-NH-$(CH_2)_4$-NH-C-$(NH_2)$=N-$SO_2$-<o>-$OCH_2$-CO-[SP] was carried out in a stepwise manner on a Beckman 990 synthesiser starting with the appropriate Boc-$NH_2$-$(CH_2)_4$-NH-C-$(NH_2)$=N-$SO_2$-< o >-$OCH_2$-CO-[SP] in accordance with the procedures set forth below in Schedule A and Schedule B

Deblocking and neutralization are preferably carried out in accordance with Schedule A as follows:

| SCHEDULE A | | |
|---|---|---|
| Step | Reagent | Mixing Time (mins) |
| 1. | Deprotect: 50% TFA in DCM | 5 |
| 2. | Deprotect: 50% TFA in DCM | 25 |
| 3. | DCM wash | 2 |
| 4. | 2-Propanol wash | 1 |
| 5. | Neutralization: 10% TEA in DCM | 2 |
| 6. | MeOH wash | 1 |
| 7. | Neutralization: 10% TEA in DCM | 2 |
| 8. | MeOH wash | 1 |
| 9. | Neutralization: 10% TEA in DCM | 2 |
| 10. | MeOH wash | 1 |
| 11. | DCM wash (three times) | 2 |

The couplings are preferably carried out as set out in Schedule B which follows:

| SCHEDULE B | | |
|---|---|---|
| Step | Reagent | Mixing Time (mins) |
| 1. | Coupling: Boc-amino acid in DCM or DMF depending on the solubility of the particular protected amino acid, plus DIC | 60-90 |
| 2. | MeOH (or DMF then MeOH) wash | 2 |
| 3. | DCM wash | 2 |
| 4. | MeOH wash | 2 |
| 5. | DCM wash | 2 |
| 6. | MeOH wash | 2 |
| 7. | DCM wash | 2 |

Briefly, BOC is used for the alpha-amino protection. Benzyl ether is used as the hydroxyl side chain protecting group for Ser and Thr. The phenolic hydroxyl group of Tyr is protected with DCB and the side chain carboxyl group of Asp is protected in the form of the cyclohexyl ester. Tos is used to protect the guanidino group of Arg and 2-Cl-Z is used as the protecting group for the Lys side-chain.

Three equivalent of Boc-protected amino acid and three equivalent of DIC are generally used in DCM. When Boc-Arg(Tos) or Boc-Lys(2-Cl-Z) is being coupled, 50% DMF/DCM is used. Boc-Ala, Boc-Ile, Boc-Leu and Boc-Nle are coupled via symmetrical anhydrides, and the HOBt ester method is used for coupling of Boc-Gln and Boc-Asn.

The following compound is obtained:

3-(4-hydroxyphenyl)propionyl-$Ala_2$-$Asp_3$($X^3$)-$Ala_4$-$Ile_5$-$Phe_6$-$Thr_7$($X^7$)-$Asn_8$-$Ser_9$($X^7$)-$Tyr_{10}$($X^{10}$)-$Arg_{11}$($X^{11}$)-$Lys_{12}$($X^{12}$)-$Val_{13}$-$Leu_{14}$-$Gly_{15}$-$Gln_{16}$-$Leu_{17}$-$Ser_{18}$($X^7$)-$Ala_{19}$-$Arg_{20}$($X^{11}$)-$Lys_{21}$($X^{12}$)-$Leu_{22}$-$Leu_{23}$-$Gln_{24}$-$Asp_{25}$-($X^3$)-$Ile_{26}$-$Nle_{27}$-$Ser_{28}$($X^7$)-NH-$(CH_2)_4$-NH-C($NH_2$) = N-$SO_2$-<o>$OCH_2$-$OCH_2$-CO-[SP] therein $X^3$ is O-cyclohexylester, $X^7$ is O-benzyl ether, $X^{10}$ is O-2,6-ClBz ether, $X^{11}$ is tosyl, $X^{12}$ is 2-Cl-Z.

In order to cleave and deprotect the protected peptide-resin, it is treated with 1 ml. m-cresol and 10 ml. hydrogen fluoride (HF) per gram of peptide resin, at 0°C for 45 min. After elimination of the HF under high vacuum, the peptide-resin remainder is washed with dry diethyl ether and ethyl acetate. The peptide is then extracted with 50% aqueous acetic acid, separated from the resin by filtration, and lyophilized.

Crude peptide is purified using either a Beckman Prep-350 preparative HPLC system (with a Beckman Type 163 variable wavelength UV detector) or a Beckman semipreparative HPLC system (with a Beckman 420 Gradient Controller, two 114M Solvent Delivery Modules, a 165 Variable Wavelength Detector and a Kipp and Zonen BD41 Recorder). In the preparative system, separations are achieved on a 41.5 x 150 mm. DYNAMAX column packed with spherical $C_{18}$ silica gel (300 A pore size, 12 um Particle size) (RAININ Inc. Co., Woburn, MA). In the semiprepara tive system, purification can be performed on a VYDAC C18

reversed phase column (10 x 250 mm., 300 A pore size, 5 um particle size).

Gradient elution is used. Solvent A consists of 0.1% aqueous TFA and solvent B is 0.1% TFA in 70% aqueous acetonitrile. The column eluate is monitored at 220 nm and 280 nm.

The peptide is judged to be substantially (>95%) pure by using a Hewlett-Packard HP-1090 liquid chromatograph. The peptides are chromatographed on a 4.6 x 250 mm. W-Porex 5 um C18 column (PHenomenex, Rancho Palos Verdes, CA) at a flow rate of 1.2 ml./min. with a mixture of Solvent A and Solvent B using a gradient (40-70% B in 30 min). The hGHRH analog obtained has HPLC retention time of 20.1 min.

## EXAMPLE IX

The synthesis of an hGHRH analog of the formula:
H-Tyr-D-$Ala_2$-$Asp_3$-$Ala_4$-$Ile_5$-$Phe_6$-$Thr_7$-$Asn_8$-$Ser_9$-$Tyr_{10}$-$Arg_{11}$-$Lys_{12}$-$Val_{13}$-$Leu_{14}$-$Gly_{15}$-$Gln_{16}$-$Leu_{17}$-$Ser_{18}$-$Ala_{19}$-$Arg_{20}$-$Lys_{21}$-$Leu_{22}$-$Leu_{23}$-$Gln_{24}$-$Asp_{25}$-$Ile_{26}$-$Nle_{27}$-$Ser_{28}$-NH-$(CH_2)_4$-NH-C($NH_2$)=NH was carried out in a stepwise manner on a Beckman 990 synthesiser starting with the appropriate Fmoc-NH-$(CH_2)_4$-NH-C-($NH_2$)=N-$SO_2$-<o>($M_s$) - $OCH_2$-CO-[SP] in accordance with the procedures set forth below in Schedules C and D.

Deblocking is carried out in accordance with Schedule C as follows:

| SCHEDULE C | | |
|---|---|---|
| Step | Reagent | Mixing Time (mins) |
| 1. | DCM wash (two times) | 3 |
| 2. | DMF wash (two times) | 3 |
| 3. | Deprotect: 20% Piperidine in DMF | 5 |
| 4. | Deprotect: 20% Piperidine in DMF | 15 |
| 5. | DMF wash (two times) | 3 |
| 6. | Dioxane/water (2:1) wash (two times) | 10 |
| 7. | DMF wash (two times | 5 |
| 8. | DCM (or DMF) wash (three times) | 5 |

Coupling is carried out in accordance with Schedule D as follows:

| SCHEDULE D | | |
|---|---|---|
| Step | Reagent | Mixing Time (mins) |
| 1 | Coupling: Fmoc-amino acid (4-6 equ) in DCM or DCM/DMF plus DIC (2-3 equ) | 60-90 |
| 2 | DMF wash (two times | 5 |
| 3 | MeOH wash (two times) | 3 |
| 4 | DCM wash (three times) | 3 |

Briefly, 4-6 equiv. Fmoc-protected amino acid and 2-3 equiv. DIC were reacted in DCM or DCM/DMF depending on the solubility of the particular protected amino acid. Tert-Butyl ether is used as the hydroxyl side-chain protecting group for Ser, Thr and Tyr. Boc is used as the protecting group for the Lys side chain and the amino group of Tyr, 4-MeO-2,3,6-tribenzene sulfonyl is used to protect the guanidino group of Arg and the Asp side-chain carboxyl group protected with t-butyl ester. Tyr is similarly protected with t-butyl ether.

The following compound is thus obtained:
$X^1$-$Tyr_1$($X^{10}$)-D-$Ala_2$-$Asp_3$($X^3$)-$Ala_4$-$Ile_5$-$Phe_6$-$Thr_7$($X^7$)-$Asn_8$($X^7$)-$Ser_9$($X^7$)-$Tyr_{10}$($X^{10}$)-$Arg_{11}$($X^{11}$)-$Lys_{12}$($X^{12}$)-$Val_{13}$-$Leu_{14}$-$Gly_{15}$-$Gln_{16}$-$Leu_{17}$-$Ser_{18}$($X^7$)-$Ala_{19}$-$Arg_{20}$($X^{11}$)-$Lys_{21}$($X^{12}$)-$Leu_{22}$-$Leu_2$$Ile_{26}$-$Nle_{27}$-$Ser_{28}$($X^7$)-NH-

$(CH_2)_4$-NH-C-$(NH_2)$ = N-$SO_2$-<o>($M_s$)-$OCH_2$-CO-[SP] wherein $X^3$ is t-Butyl ester, $X^7$ is t-butyl ether, $X^{10}$ is t-butyl ether, $X^{11}$ is 4-methoxy-2,3,6-trimethylbenzylsulfonyl, $X^1$ and $X^{12}$ are Boc.

The protected peptide produced as above is utilized as starting material. The cleavage of the peptide from the solid support and the removal of side chain protecting groups was carried out simultaneously by the reaction of the protected peptide resin with TFA-thioanisole (5%) at a temperature 25-30°C for 2-3 hours (10 ml. of liquid per each gram of resin was used). After the completion of the reaction the reagent was removed in vacuo at room temperature. The remaining material was extracted, first with ether (removing the nonpolar side-products), then the crude peptide was extracted from the filtered-cake with 50% acetic acid and the filtrate was lyophilized.

## EXAMPLE X

The synthesis of an hGHRH analog of the formula:
3-(4-hydroxyphenyl)propionyl-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Ser$_{18}$-Ala$_{19}$-Arg$_{20}$-Lys$_{21}$-Leu$_{22}$ Leu$_{23}$-Gln$_{24}$-Asp$_{25}$-Ile$_{26}$-Nle$_{27}$-Ser$_{28}$-NH-$(CH_2)_4$-NH-C-$(NH_2)$ = NH was carried out as described in Example VIII, with the exception that Boc-D-Lys(2-Cl-Z) is incorporated in place of Boc-Lys(2-Cl-Z) in position 12 to give another protected peptide-resin having the formula
3-(4-hydroxyphenyl)propionyl-Ala$_2$-Asp$_3$(OChx)-Ala$_4$-Ile$_5$-Phe$_6$-Thr$_7$(Bzl)-Asn$_8$-Ser$_9$(Bzl)-Tyr$_{10}$(DCB)-Arg$_{11}$-(Tos)-D-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-Lys$_{21}$(2-Cl-Z)-Leu$_{22}$-Leu$_{23}$-Gln$_{24}$-Asp$_{25}$(OChx)-Ile$_{26}$-Nle$_{27}$-Ser$_{28}$(Bzl)-NH-$(CH_2)_4$-NH-C$(NH_2)$ = N-[SPA]-[SP],
which is then similarly converted to the desired peptide in accordance with the procedure of Example IX. The hGHRH analog thus obtained has HPLC retention time of 19.2 min.

## EXAMPLE XI

The synthesis of an hGHRH analog of the formula:
3-(4-hydroxyphenyl)propionyl-Ala$_2$-Asp$_3$-Ala$_4$-Ile$_5$-Phe$_6$-Thr$_7$-Asn$_8$-Ser$_9$-Tyr$_{10}$-Arg$_{11}$-Lys$_{12}$-Val$_{13}$-Leu$_{14}$-Ala$_{15}$-Gln$_{16}$-Leu$_{17}$-Ser$_{18}$-Ala$_{19}$-Arg$_{20}$-D-Lys$_{21}$-Leu$_{22}$-Leu$_{23}$-Gln$_{24}$-Asp$_{25}$-Ile$_{26}$-Nle$_{27}$-Ser$_{28}$-NH-$(CH_2)_4$-NH-C-$(NH_2)$ = NH was carried out as described in Example VIII, with the exception that Boc-D-Lys(2-Cl-Z) is incorporated in place of Boc-Lys(2-Cl-Z) in position 21 to give another protected peptide-resin having the formula
3-(4-hydroxyphenyl)propionyl-Ala$_2$-Asp$_3$(OChx)-Ala$_4$-Ile$_5$-Phe$_6$-Thr$_7$-(Bzl)-Asn$_8$-Ser$_9$(Bzl)-Tyr$_{10}$(DCB)-Arg$_{11}$-(Tos)-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-D-Lys$_{21}$(2-Cl-Z)-Leu$_{22}$-Leu$_{23}$-Gln$_{24}$-Asp$_{25}$(OChx)-Ile$_{26}$-Nle$_{27}$-Ser$_{28}$(Bzl)-NH-$(CH_2)_4$-NH-C$(NH_2)$ = N-[SPA]-[SP],
which is then similarly converted to the desired peptide in accordance with the procedure of Example VIII. The hGHRH analog thus obtained has HPLC retention time of 19.6 min.

## EXAMPLE XII

The synthesis of an hGHRH analog of the formula:
3-(4-hydroxyphenyl)propionyl-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Ser$_{18}$-Ala$_{19}$-Arg$_{20}$-D-Lys$_{21}$-Leu$_{22}$-Leu$_{23}$-Gln$_{24}$-Asp$_{25}$- Ile$_{26}$-Nle$_{27}$-Ser$_{28}$-$(CH_2)_4$-NH-C-$(NH_2)$ = NH was carried out as described in Example VIII, with the exception that Boc-D-Lys(2-Cl-Z) is incorporated in place of Boc-Lys(2-Cl-Z) in positions 12 and 21 to give another protected peptide-resin having the formula
3-(4-hydroxyphenyl)propionyl-Ala$_2$-Asp$_3$(OChx)-Ala$_4$-Ile$_5$-Phe$_6$-Thr$_7$(Bzl)-Asn$_8$-Ser$_9$(Bzl)-Tyr$_{10}$(DCB)-Arg$_{11}$-(Tos)-D-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-D-Lys$_{21}$(2-Cl-Z)-Leu$_{22}$-Leu$_{23}$-Gln$_{24}$-Asp$_{25}$(OChx)-Ile$_{26}$-Nle$_{27}$-Ser$_{28}$(Bzl)-NH-$(CH_2)_4$-NH-C$(NH_2)$ = N-[SPA]-[SP],
which is then similarly converted to the desired peptide in accordance with the procedure of Example VIII. The hGHRH analog thus obtained has HPLC retention time of 17.9 min.

## EXAMPLE XIII

The synthesis of an hGHRH analog of the formula:
3-(4-hydroxyphenyl)propionyl-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Se$_{10}$-Ala$_{19}$-Arg$_{20}$-Lys$_{21}$-D-Leu$_{22}$-Leu$_{23}$-Gln$_{24}$-Asp$_{25}$-Ile$_{26}$-Nle$_{27}$-Ser$_{28}$-NH-$(CH_2)_4$-NH-C-$(NH)_2$ = NH is carried out as described in Example VIII, with the exception that Boc-D-Leu is incorporated in place of Boc-Leu in position 22, and that Boc-D-Lys(2-Cl-Z) is incorporated in place of Boc-Lys(2-Cl-Z) in position 12 to give another protected peptide-resin having the formula:
3-(4-hydroxyphenyl)propionyl-Ala$_2$-Asp$_3$(OChx)-Ala$_4$-Ile$_5$-Phe$_6$-Thr$_7$(Bzl)-Asn$_8$-Ser$_9$(Bzl)-Tyr$_{10}$(DCB)-Arg$_{11}$-(Tos)-D-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-Lys$_{21}$(2-Cl-Z)-D-Leu$_{22}$-Leu$_{23}$-Gln$_{24}$-Asp$_{25}$(OChx)-Ile$_{26}$-Nle$_{27}$-Ser$_{28}$(Bzl)-NH-$(CH_2)_4$-NH-C$(NH_2)$ = N-[SPA]-[SP],
which is then similarly converted to the desired peptide in accordance with the procedure of Example VIII. The hGHRH analog thus obtained has HPLC retention time of 17.9 min.

## EXAMPLE XIV

The synthesis of an hGHRH analog of the formula:
3-(4-hydroxyphenyl)propionyl-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Ser$_{10}$-Ala$_{19}$-Arg$_{20}$-Lys$_{21}$-Leu$_{22}$-D-Leu$_{23}$-Gln$_{24}$-Asp$_{25}$-Ile$_{26}$-Nle$_7$-Ser$_{28}$-NH-$(CH_2)_4$-NH-C-$(NH)_2$ = NH is carried out as described in Example VIII, with the exception that Boc-D-Leu is incorporated in place of Boc-Leu in position 23, and that Boc-D-Lys(2-Cl-Z) is incorporated in place of Boc-Lys(2-Cl-Z) in position 12 to give another protected peptide-resin having the formula:
3-(4-hydroxyphenyl)propionyl-Ala$_2$-Asp$_3$(OChx)-Ala$_4$-Ile$_5$-Phe$_6$-Thr$_7$(Bzl)-Asn$_8$-Ser$_9$(Bzl)-Tyr$_{10}$(DCB)-Arg$_{11}$-(Tos)-D-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-Lys$_{21}$(2-Cl-Z)-Leu$_{22}$-D-Leu$_{23}$-Gln$_{24}$-Asp$_{25}$(OChx)-Ile$_{26}$-Nle$_{27}$-Ser$_{18}$(Bzl)-NH-$(CH_2)_4$-NH-C$(NH_2)$ = N-[SPA]-[SP],
which is then similarly converted to the desired peptide in accordance with the procedure of Example VIII. The hGHRH analog thus obtained has HPLC retention time of 17.6 min.

## EXAMPLE XV

The synthesis of an hGHRH analog of formula:
2-carboxybenzoyl-Gly-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Ser$_{10}$-Ala$_{19}$-Arg$_{20}$-D-Lys$_{21}$-Leu$_{22}$-Leu$_{23}$-Gln$_{24}$-Asp$_{25}$-Ile$_{26}$-Nle$_{27}$-Ser$_{28}$-NH-$(CH_2)_4$-NH-C-(NH)-$_2$ = NH is carried out as described in Example VIII, with the exception that Boc-D-Lys(2-Z-Cl) is incorporated in place of Boc-Lys(2-Z-Cl) in positions 12 and 21, that Boc-Gly is coupled instead of 3-(4-hydroxyphenyl)-propionyl to the N-terminus, and that the Gly residue, after removing the Boc group, is reacted with phthalic anhydride to give another protected peptide-resin having the formula
2-carboxybenzoyl-Gly-Ala$_2$-Asp$_3$(OChx)-Ala$_4$-Ile$_5$-Phe$_6$-Thr$_7$(Bzl)-Asn$_8$-Ser$_9$(Bzl)-Tyr$_{10}$(DCB)-Arg$_{11}$(Tos)-D-Lys$_{12}$(2-Cl-Z)-Val$_{13}$-Leu$_{14}$-Ala$_{15}$-Gln$_{16}$-Leu$_{17}$-Ser$_{18}$(Bzl)-Ala$_{19}$-Arg$_{20}$(Tos)-D-Lys$_{21}$(2-Cl-Z)-Leu$_{22}$-Leu$_{23}$-Gln$_{24}$-Asp$_{25}$(OChx)-Ile$_{26}$-Nle$_{27}$-Ser$_{28}$(Bzl)-NH-$(CH_2)_4$-NH-C$(NH_2)$ = N-[SPA]-[SP],
which is then similarly converted to the desired peptide in accordance with the procedure of Example VIII. The hGHRH analog thus obtained has HPLC retention time of 15.5 min.

## EXAMPLE XVI

The synthesis of an hGHRH analog of formula:
Glutaryl-D-Ala$_2$-Asp$_3$-Ala$_4$-Ile$_5$-Phe$_6$-Thr$_7$-Asn$_8$-Ser$_9$-Tyr$_{10}$-Arg$_{11}$-D-Lys$_{12}$-Val$_{13}$-Leu$_{14}$-Ala$_{15}$-Gln$_{16}$-Leu$_{17}$-Ser$_{10}$-Ala$_{19}$-Arg$_{20}$-D-Lys$_{21}$-Leu$_{22}$-Leu$_{23}$-Gln$_{24}$-Asp$_{25}$-Ile$_{26}$-Nle$_{27}$-Ser$_{28}$-NH-$(CH_2)_4$-NH-C-$(NH)_2$ = NH is carried out as described in Example VIII, with the exception that Boc-D-Ala is incorporated in place of Boc-Ala in position 2 and after removing the Boc group, is reacted with glutaric anhydride to give another protected peptide-resin having the formula

Glutaryl-D-$Ala_2$-$Asp_3$(OChx)-$Ala_4$-$Ile_5$-$Phe_6$-$Thr_7$(Bzl)-$Asn_8$-$Ser_9$(Bzl)-$Tyr_{10}$(DCB)-$Arg_{11}$(Tos)-D-$Lys_{12}$(2-Cl-Z)-$Val_{13}$-$Leu_{14}$-$Ala_{15}$-$Gln_{16}$-$Leu_{17}$-$Ser_{18}$(Bzl)-$Ala_{19}$-$Arg_{20}$(Tos)-D-$Lys_{21}$(2-Cl-Z)-$Leu_{22}$-$Leu_{23}$-$Gln_{24}$-$Asp_{25}$-(OChx)-$Ile_{26}$-$Nle_{27}$-$Ser_{28}$(Bzl)-$NH_2$-$(CH_2)_4$-NH-C($NH_2$) = N-[SPA]-[SP], which is then similarly converted to the desired peptide in accordance with the procedure of Example VIII. The hGHRH analog thus obtained has HPLC retention time of 19.4 min.

Test Procedures

The compounds of the present invention were tested both in vitro and in vivo . Growth hormon releasing activities in vitro are summarized in Table 1. In vivo effect of the compounds on the release of GH in rats after intravenous (i.v.) and subcutaneous (sc.) injections are given in Tables 2 and 3, and Tables 4 and 5, respectively.

## EXAMPLE XVII

Growth hormone releasing activity in vitro was assayed by using a superfused rat pituitary cell system (S. Vigh and A. V. Schally, Peptides 5 , Suppl: 241-347, 1984). Each peptide, hGH-RH(1-29)$NH_2$ (as control) and hGHRH analogs of the present invention, was administered for 3 min. (1 ml. perfusate) at various concentrations as shown below. GH content of the 1 ml. fractions collected was determined by RIA.

TABLE 1

In vitro effect of hGHRH analogs on the GH release in a superfused rat pituitary cell system.

| Peptide | Test Conditions (nM) | | Potentency relative to hGHRH (1-29)$NH_2$ (=1)* |
|---|---|---|---|
| hGHRH (1-29) $NH_2$ (Control) | 2 and 10 | | 1.0 |
| Example VIII-Sample | 0.02 | 0.1 | 254 |
| Exmple X-Sample | 0.1 | 0.5 | 73.5 |
| Example XI-Sample | 0.1 | 0.5 | 33.3 |
| Example XII-Sample | 0.1 | 0.5 | 34.2 |
| Example XIV-Sample | 0.2 | 1.0 | 34.8 |

*Assumed 4-point assay

## EXAMPLE XVIII

Adult female rats were used and were anesthetized with pentobarbital (5 mg/100 g, b.w.), injected i.p.; 30 minutes after the injection of pentobarbital, blood samples were taken from the jugular vein (pretreated level) and immediately thereafter hGH-RH(1-29)$NH_2$ (as a control) or hGH-RH(1-29)$NH_2$ analogs were injected i.v. Blood samples were taken from the jugular vein 5, 15 and 30 minutes after the injection. The blood samples were centrifuged, plasma was removed and the GH level was measured by RIA.

The results are tabulated in Table 2 below and are summarized in Table 3.

Table 2

*In vivo* Effect of hGHRH analogs on the GH release in the rat after intraveneous (IV) injection.

| Peptide | Dose ug/kg | GH released (ng/ml)* 0 min. | 5 min. | 15 min. | 30 min. |
|---|---|---|---|---|---|
| EXPERIMENT I | | | | | |
| Saline | | 69±12.2 | 82±11.6 | 100±13.5 | 97±13.8 |
| $hGHRH(1-29)NH_2$ | 2.5 | 68+12.1 | 421+29.9 | 112+10.8 | 60+6.6 |
| (Control) | 1.0 | 70±11.9 | 225±29.8 | 103±13.4 | 79±9.3 |
| Example VIII- | 0.25 | 72±11.0 | 643±88.6 | 241±21.0 | 106±18.9 |
| Sample | 0.1 | 83±11.3 | 305±36.0 | 146±24.3 | 92±11.0 |
| Example X- | 0.25 | 76±10.4 | 599±61.5 | 160±13.5 | 71±10.6 |
| Sample | 0.1 | 72±11.2 | 239±30.5 | 142±30.5 | 80±21.1 |

*Mean ± SEM

TABLE 2 (continued)

| Peptide | Dose ug/kg | GH released (ng/ml)* 0 min. | 5 min. | 15 min. | 30 min. |
|---|---|---|---|---|---|
| EXPERIMENT 2 | | | | | |
| Saline | | 61±17.8 | 43±12.2 | 37±8.0 | 27±10.6 |
| $hGHRH(1-29)NH_2$ | 2.5 | 79±16.5 | 274±23.6 | 58±10.7 | 23±4.4 |
| (Control) | 1.0 | 56±11.2 | 164±14.6 | 41±7.5 | 23±11.1 |
| Example XI- | 0.5 | 60±9.0 | 485±70.4 | 121±29.6 | 26±3.8 |
| Sample | 0.2 | 48±16.3 | 248±25.4 | 58±14.7 | 28∓6.9 |
| EXPERIMENT 3 | | | | | |
| Saline | | 74±17.5 | 65±14.4 | 54±11.9 | 33±9.9 |
| $hGHRH(1-29)NH_2$ | 2.5 | 84±15 | 269±32.4 | 87±25.2 | 42±13 |
| (Control) | 1.0 | 57±12.2 | 154±7.4 | 85±19.8 | 73±36 |
| Example XII- | 0.25 | 61±8.5 | 377±50.2 | 73±8.4 | 40±16.2 |
| Sample | 0.1 | 44±11 | 153±14.7 | 75±23.3 | 72±17.7 |

*Mean ± SEM

Table 3

| Potency of hGHRH analogs of Examples VIII, X and XI relative to hGHRH (1-29)$NH_2$ (=1) as calculated from the data of Table 2 by using the 4-point assay method. | | | |
|---|---|---|---|
| hGHRH analog | After (min.) | Potency | (Range of Potency)* |
| Example VIII-Sample | 5 | 15.16 | (10.52 - 21.87) |
| | 15 | 20.96 | (12.74 - 33.46) |
| Example X-Sample | 5 | 13.48 | (10.38 - 17.51) |
| | 15 | 26.92 | (8.50 - 85.21) |
| Example XI-Sample | 5 | 10.62 | (5.92 - 19.07) |
| | 15 | 12.44 | (3.51 - 44.06) |
| Example XII-Sample | 5 | 16.68 | (11.40 - 24.40) |

*Confidence limit: 95%.

## EXAMPLE XIX

Adult female rats were used and were anesthetized with pentobarbital (5 mg/100 g, b.w.), injected i.p.; 20 minutes after the injection of pentobarbital, blood samples were taken from the jugular vein (pretreated level) and immediately thereafter hGH-RH(1-29)$NH_2$ (as a control) or hGH-RH analogs were injected i.v. Blood samples were taken from the jugular vein 15, 30 and 60 minutes after the injection. The blood samples were centrifuged, plasma was removed and the GH level was measured by RIA.

The results are tabulated in Table 4 below and are summarized in Table 5.

Table 4

| *In vivo* effect of hGHRH analogs on the GH release in the rat after subcutaneous (SC) injection | | | | | |
|---|---|---|---|---|---|
| | Dose | GH released (ng/ml)* | | | |
| Peptide | ug/kg | 0 min. | 15 min. | 30 min. | 60 min. |
| Saline | | 79±16.4 | 84±13.4 | 86±4.1 | 74±19.5 |
| hGHRH(1-29)$NH_2$ | 250 | 57±21.0 | 269±30.3 | 232±37.0 | 131±33.9 |
| (Control) | 100 | 71±5.1 | 173±19.8 | 184±29.2 | 71±15.5 |
| Example VIII-Sample | 2.5 | 94±12.0 | 375±76.5 | 197±21.4 | 94±17.7 |
| | 1.0 | 99±11.2 | 178±18.4 | 132±15.1 | 65±16.5 |
| Example XII-Sample | 2.5 | 92+5.0 | 251±48.0 | 116±11.5 | 56±16.5 |
| | 1.0 | 71±16.6 | 248±96.6 | 166±75.0 | 112±7.4 |

*Mean ± SEM

Table 5

| Potency of hGHRH Analogs of Example VIII and IX, Relative to hGHRH(1-29)$NH_2$(=1) as Calculated from the Data of Table 4 by Using the 4-Point Assay Method. | | | |
|---|---|---|---|
| hGHRH analog | After (min.) | Potency | (Range of Potency)* |
| Example VIII-Sample | 15 | 158.94 | (87.45 - 288.87) |
| | 30 | 133.98 | (63.57 - 282.39) |
| Example XII-Sample | 15 | 162.48 | (70.78 - 372.98) |
| | 30 | 247.25 | (31.07 - 1967.6) |

*Mean ± SEM

## Claims

1. A polypeptide of the structure :

$Q^1$-CO-$R_2$-$R_3$-$Ala_4$-$Ile_5$-$Phe_6$-$Thr_7$-$R_8$-$Ser_9$-$R_{10}$-$Arg_{11}$-$R_{12}$-$R_{13}$-$R_{14}$-$R_{15}$-$Gln_{16}$-$R_{17}$-$R_{18}$-$Ala_{19}$-$Arg_{20}$-$R_{21}$-$R_{22}$-$R_{23}$-$R_{24}$-$R_{25}$-$Ile_{26}$-$R_{27}$-$R_{28}$-NH-$Q^2$

I.

wherein $Q^1$ is 3-carboxypropyl, 2-carboxybenzamido methyl, an omega or alpha-omega substituted alkyl of the structure:

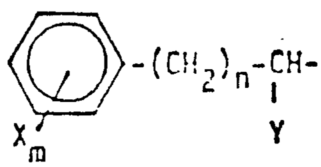

II.

where

X=OH

Y=H,OH,$NH_2$,$CH_3$CONH,

m= 1,2

n= 0,1,2

$R_2$ is Ala or D-Ala,

$R_3$ is Asp,D-Asp,Glu,or D-Glu

$R_8$ is Asn,D-Asn,Ser, or D-Ser

$R_{10}$ is Tyr or D-Tyr

$R_{12}$ is Lys, D-Lys, Arg, Orn, D-Arg, or D-Orn

$R_{13}$ is Val or Ile

$R_{14}$ is Leu or D-Leu

$R_{15}$ is Ala

$R_{17}$ is Leu or D-Leu

$R_{18}$ is Tyr or Ser

$R_{21}$ is Lys, D-Lys, Orn, D-Orn, Arg or D-Arg

$R_{22}$ is Leu, D-Leu or Ala

$R_{23}$ is Leu or D-Leu

$R_{24}$ is Gln or His

$R_{25}$ is Asp,D-Asp,Glu, or D-Glu

$R_{27}$ is Met,D-Met,Ala,Nle,Ile,Val,Nva,Leu

$R_{28}$ is Asn or Ser

$Q^2$ is a lower omega-guanidino-alkyl group having a formula:

$$-(CH_2)_p-NH-C-NH_2$$

"

NH III

wherein p = 2 - 6
and the pharmaceutically acceptable salts addition thereof with the pharmaceutically acceptable organic or inorganic bases and organic or inorganic acids.

2. A product of Claim 1 wherein p is 4.
3. A product of Claim 2 wherein $Q^1$ is 2-(4-hydroxyphenyl)ethyl, $R_2$ and $R_{15}$ are Ala, and $R_{27}$ is Nle.
4. A product of Claim 3 wherein $R_{12}$, $R_1$ and/or $R_{21}$ is D-Lys.
5. A product of Claim 4 wherein $R_{22}$ or $R_{23}$ is D-Leu, if $R_{12}$ is D-Lys.
6. A product of Claim 4 wherein $Q^1$ is 2-carboxybenzamidomethyl, if $R_1$ and $R_{21}$ are D-Lys.
7. A method of anchoring the $Q^2$ segment of $NH_2$-$Q^2$ moiety as defined in Claim 1 to a support phase [SP] selected from the group consisting of amino type resins which comprises either in a first feature the sequential steps of:
reacting t-butoxycarbonylating agent with $NH_2.Q^2$ in the presence of a base to form Boc-NH-$Q^{2,}$
reacting said Boc-NH-$Q^2$ with an arylsulphonyl halide of the formula:
Hal-$SO_2$ -< o >-O-$CH_2$-COOH
wherein Hal is chloro or bromo, at between -5 and 10°C in a water soluble ether in the presence of aqueous alkali to form the corresponding
Boc- NH-$Q^{2'}$-$SO_2$-< o >-$OCH_2$- COOH
which is coupled to the support phase [SP] by the action of a diloweralkyl carbodiimide to yield:
Boc-NH-$Q^{2'}$-$SO_2$-< o >-O-$CH_2$-CO- [SP]
wherein $Q^{2'}$ is

$$-(CH_2)_p-NH-C-NH-$$

"

NH IV

or in a second feature the sequential steps of:
reacting benzyloxy carbonyl chloride with $NH_2$-$Q^2$ in in presence of a base to form Cbz-NH-$Q^2$,
reacting said Cbz-NH-$Q^2$ with a substituted arylsulphonyl chloride of the formula:

O

"

Hal-S- < o >-O-$CH_2$-COOH

" /

O $M_s$

wherein Hal is chloro or bromo , M is lower alkyl or lower alkoxy of 1 to 5 carbon atoms and is 1-3, in the presence of a strong aqueous base and removing the Cbz group by catalytic hydrogenation to yield

HNH-$Q^{2'}$ -$SO_2$-< o >-$OCH_2$- COOH

/

$M_s$

reacting this product with 9-fluorenylmethoxy carbonyl chloride in the presence of a base to yield

$$Fmoc\text{-}NH\text{-}Q^{2'}\text{-}SO_2\text{-}\lt\ o\ \gt\text{-}OCH_2\text{-}COOH$$

/

$M_s$

where s = 1-3
coupling this product to said support phase [SP] to yield:

O

"

$$Fmoc\text{-}NH\text{-}Q^{2'}\text{-}S\text{-}\lt\ o\ \gt\text{-}O\text{-}CH_2\text{-}CO\text{-}\ [SP]$$

" /

O $M_s$

8. A method of Claim 9 wherein the support phase [SP] is selected from the group consisting of amino resins of the formula
$H_2N\text{-}CH_2\text{-}\lt o \gt\text{-}[Pm]$
to yield in the first feature
$Boc\text{-}NH\text{-}Q^{2'}\text{-}SO_2\text{-}\lt o \gt\text{-}O\text{-}CH_2\text{-}CO\text{-}NH\text{-}CH_2\ \text{-}\lt o \gt\text{-}\ [Pm]$
and to yield in the second feature

$$Fmoc\text{-}NH\text{-}Q^{2'}\text{-}SO_2\text{-}\lt\ o\ \gt\text{-}O\text{-}CH_2\text{-}CO.NH.CH_2\text{-}\lt\ o\ \gt\text{-}[Pm]$$

/

$M_s$

where [Pm] is the polymeric portion of the support phase.

9. A method of cleaving a protected peptide [PR][PeP] attached to a support phase [SP] selected from the group consisting of amino type resins, said combination having either the first structure:

$$[PR][PeP]\ \text{-}SO_2\text{-}\lt\ o\ \gt\text{-}O\text{-}CH_2\text{-}CO\text{-}\ [SP]$$

/

$M_s$

where [PeP] has the structure
$Q_1\text{-}CO\text{-}R_2\text{-}R_3\text{-}Ala_4\text{-}Ile_5\text{-}Phe_6\text{-}Thr_7\text{-}R_8\text{-}Ser_9\text{-}R_{10}\text{-}Arg_{11}\text{-}R_{12}\text{-}R_{13}\text{-}R_{14}\text{-}R_{15}\text{-}Gln_{16}\text{-}R_{17}\text{-}R_{18}\text{-}Ala_{19}\text{-}Arg_{20}\text{-}R_{21}\text{-}R_{22}\text{-}R_{23}\text{-}R_{24}\text{-}R_{25}\text{-}Ile_{26}\text{-}R_{27}\text{-}R_{28}\text{-}NH\text{-}Q^{2'}$
wherein M is methyl or methoxy and s is 1 - 3, [PR] signifies side chain carboxylic groups protected by t-butyl ester groups and whose phenolic and alcoholic side chains are by trifluoroacetic acid sensitive groups selected from the group consisting to t-butyl ethers and amino groups protected by t-butoxycarbonyl and guanidino side chains protected by substituted benzenesulfonyl groups, $Q^1$ is 3-carboxypropyl or 2-carboxybenzamidomethyl group, and omega or alpha-omega substituted alkyl of the structure:

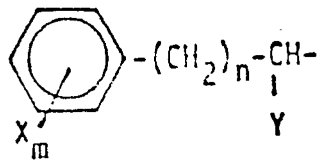

where:
X = OH
Y = H,OH,$NH_2$,$CH_3CONH$,
m = 1,2
n = 0,1,2
$R_2$ is Ala or D-Ala
$R_3$ is Asp, D-Asp, Glu or D-Glu
$R_8$ is Asn, D-Asn, Ser or D-Ser
$R_{10}$ is Tyr or D-Tyr
$R_{12}$ is Lys, D-Lys, Arg, Orn, D-Arg or D-Orn
$R_{13}$ is Val or Ile
$R_{14}$ is Leu or D-Leu
$R_{15}$ is Ala
$R_{17}$ is Leu or D-Leu
$R_{18}$ is Tyr or Ser
$R_{21}$ is Lys, D-Lys, Orn, D-Orn, Arg or D-Arg
$R_{22}$ is Leu, D-Leu or Ala
$R_{23}$ is Leu or D-Leu
$R_{24}$ is Gln or His
$R_{25}$ is Asp, D-Asp, Glu or D-Glu
$R_{27}$ is Nle,
$R_{28}$ is Asn or Ser
$Q^{2'}$ is a lower omega-guanidino-alkyl group having a formula:

```
-(CH )  -NH-C-NH
    2 p       ‖   2
             NH
```

wherein p = 2 - 6
which comprises treating the protected peptide with trifluoracetic acid,
or the second structure

```
[PR][PeP] -SO -< o >-O-CH -CO- [SP]
             2            2
                /
               M
```

where [PeP] has the structure
$Q^2$-CO-$R_2$-$R_3$-$Ala_4$-$Ile_5$-$Phe_6$-$Thr_7$-$R_8$-$Ser_9$-$R_{10}$-$Arg_{11}$-$R_{12}$-$R_{13}$-$R_{14}$-$R_{15}$-$Gln_{16}$-$R_{17}$-$R_{18}$-$Ala_{19}$-$Arg_{20}$-$R_{21}$-$R_{22}$-$R_{23}$-$R_{24}$-$R_{25}$-$Ile_{26}$-$R_{27}$-$R_{28}$-NH-$Q^{2'}$
wherein M is hydrogen, [PR] signifies side chain carboxylic groups protected by benzylic ester or cycloalkyl ester groups and whose phenolic and alcoholic side chains are by trifluoroacetic acid resistant groups selected from the group consisting of benzylic or substituted benzylic ethers and amino side chains protected by benzyloxycarbonyl or halobenzyloxycarbonyl groups and guanidino side chains protected by p-toluene sulfonyl groups, $Q^1$ is a 3-carboxypropyl or 2-carboxybenzamidomethyl group, an omega or alpha-omega substituted alkyl of the structure:

$$-(CH_2)_n-\underset{\displaystyle Y}{CH}-$$

where:
X = OH
Y = H,OH,$NH_2$,$CH_3CONH$
m = 1,2
n = 0,1,2
$R_2$ is Ala or D-Ala
$R_3$ is Asp, D-Asp, Glu or D-Glu
$R_8$ is Asn, D-Asn, Ser or D-Ser
$R_{10}$ is Tyr or D-Tyr
$R_{12}$ is Lys, D-Lys, Arg, Orn, D-Arg or D-Orn
$R_{13}$ is Val or Ile
$R_{14}$ is Leu or D-Leu
$R_{15}$ is N-Methyl-Gly or Ala
$R_{17}$ is Leu or D-Leu
$R_{18}$ is Tyr or Ser
$R_{21}$ is Lys, D-Lys, Orn D-Orn, Arg or D-Arg
$R_{22}$ is Leu, D-Leu or Ala
$R_{23}$ is Leu or D-Leu
$R_{24}$ is Gln or His
$R_{25}$ is Asp, D-Asp, Glu or D-Glu
$R_{27}$ is Nle,
$R_{28}$ is Asn or Ser
$Q^{2'}$ is a lower omega-guanidino-alkyl group having a formula:

$$-(CH_2)_p-NH-\underset{\displaystyle \overset{\|}{NH}}{C}-NH_2$$

wherein p = 2 - 6
which comprises treating the protected peptide with hydrofluoric acid.

10. A method of Claim 9 wherein the support phase [SP] is selected from the group consisting of amino resins of the formula
$H_2N$-$CH_2$-< o >-[Pm]
where [Pm] is the polymeric portion of the support phase.

11. A process of Claim 9 wherein p is 4.

12. Use of a compound of claim 1 in a physiologically acceptable carrier for enhacing milk production in females of milk producing mammals.

European Patent Office

# EUROPEAN SEARCH REPORT

Application Number

EP 89 11 5466

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 320 785 (HOECHST)<br>* Pages 2-3, (in full) page 6, lines 10-49; claims * | 1-2,7-12 | C 07 K 7/10<br>A 61 K 37/02 |
| Y | EP-A-0 277 626 (TULANE EDUCATIONAL FUND)<br>* Claims * | 1-2,7-12 | |
| Y | EP-A-0 264 750 (HOFFMAN-LA ROCHE)<br>* Column 1, lines 36-52; column 2, line 28 - column 3, line 38; claims * | 1-2,7-12 | |
| Y | LIFE SCIENCES, vol. 42, no. 1, 1988, pages 27-35, New York, US; KOVOCS et al.: "An evaluation of intravenous, subcutaneous, and in vitro activity of new agmatine analogs of growth hormone-releasing hormone hgH-RH(1-29)NH2"<br>* Pages 27-28 (until "in vitro assays") page 30 (Table 1) * | 1-2,7-12 | |

TECHNICAL FIELDS SEARCHED (Int. Cl.5)

C 07 K
A 61 K

The present search report has been drawn up for all claims

| Place of search | Date of completion of the search | Examiner |
|---|---|---|
| THE HAGUE | 07-03-1990 | KORSNER S.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document